(19) Europäisches Patentamt / European Patent Office / Office européen des brevets

(11) **EP 3 167 285 B1**

(12) **FASCICULE DE BREVET EUROPEEN**

(45) Date de publication et mention de la délivrance du brevet:
**25.04.2018 Bulletin 2018/17**

(21) Numéro de dépôt: **15735965.4**

(22) Date de dépôt: **09.07.2015**

(51) Int Cl.:
*G01N 33/08* (2006.01)     *G01N 21/31* (2006.01)
*G01N 21/65* (2006.01)     *G01N 21/552* (2014.01)

(86) Numéro de dépôt international:
**PCT/EP2015/065778**

(87) Numéro de publication internationale:
**WO 2016/005539 (14.01.2016 Gazette 2016/02)**

(54) **DISPOSITIF NON INVASIF DE DÉTERMINATION DE LA FERTILITÉ ET/OU DU SEXE D'UN OEUF, ET PROCÉDÉ CORRESPONDANT**

VORRICHTUNG UND VERFAHREN ZUR NICHT-INVASIVEN BESTIMMUNG DER FRUCHTBARKEIT UND/ODER DES GESCHLECHTS VON EIERN

NON-INVASIVE DEVICE AND METHOD FOR DETERMINING FERTILITY AND/OR SEX OF AN EGG

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.07.2014 FR 1456744**

(43) Date de publication de la demande:
**17.05.2017 Bulletin 2017/20**

(73) Titulaire: **Tronico**
**85660 Saint Philbert de Bouaine (FR)**

(72) Inventeur: **SCHORTGEN, Marc**
**F-35113 Domagne (FR)**

(74) Mandataire: **Vidon Brevets & Stratégie**
**16B, rue de Jouanet**
**B.P. 90333**
**Technopole Atalante**
**35703 Rennes Cedex 7 (FR)**

(56) Documents cités:
**WO-A2-2014/021715     US-A1- 2002 075 476**
**US-A1- 2013 044 210     US-B2- 8 624 190**

- L. LIU ET AL: "Detecting Fertility and Early Embryo Development of Chicken Eggs Using Near-Infrared Hyperspectral Imaging", FOOD AND BIOPROCESS TECHNOLOGY, vol. 6, no. 9, 2 août 2012 (2012-08-02), pages 2503-2513, XP055173098, ISSN: 1935-5130, DOI: 10.1007/s11947-012-0933-3
- GERALD STEINER ET AL: "Gender determination of fertilized unincubated chicken eggs by infrared spectroscopic imaging", ANALYTICAL AND BIOANALYTICAL CHEMISTRY, SPRINGER, BERLIN, DE, vol. 400, no. 9, 9 avril 2011 (2011-04-09), pages 2775-2782, XP019909218, ISSN: 1618-2650, DOI: 10.1007/S00216-011-4941-3

**Description**

## 1. DOMAINE DE L'INVENTION

Le domaine de l'invention est celui de l'aviculture.

Plus précisément, l'invention concerne une technique non invasive de détermination de la fertilité d'un oeuf et/ou du sexe de celui-ci (c'est-à-dire le genre, mâle ou femelle, de l'embryon contenu dans l'oeuf s'il a été fécondé).

## 2. ARRIÈRE-PLAN TECHNOLOGIQUE

On connaît depuis de nombreuses années, dans le domaine avicole, la problématique récurrente de la détermination de la fertilité et du sexe des oeufs avant leur éclosion.

Traditionnellement, le sexage s'effectue sur le poussin d'un jour par une intervention humaine principalement via deux techniques : l'observation des plumes et l'observation du cloaque.

Bien qu'efficaces, un inconvénient majeur de ces deux techniques est qu'elles interviennent trop tard dans le cycle de production. Par exemple, pour les poules pondeuses, il faut 21 jours d'incubation pour séparer les femelles des mâles (ces derniers étant éliminés).

La détermination du genre des embryons à un stade précoce de l'incubation permettrait d'améliorer la production avicole, avec à la fois un gain de temps et un gain financier.

Par conséquent, un certain nombre de techniques de détermination, plus ou moins invasives, ont été développées afin d'évaluer la fertilité et le sexe d'un oeuf d'oiseau non éclot.

Une première technique connue est décrite dans le brevet US6029080, qui propose une méthode de détermination par résonance magnétique nucléaire. Cette technique permet de trier les oeufs en trois catégories: les oeufs contenant un embryon mâle, ceux contenant un embryon femelle et les oeufs clairs (non fécondés). Le tri s'effectue par l'observation, à travers la coquille, des organes reproducteurs. Plus particulièrement, l'observation n'est praticable qu'après refroidissement de l'oeuf afin de minimiser les mouvements de l'embryon. Un inconvénient de cette technique est que le refroidissement de l'oeuf engendre des perturbations dans le développement futur de l'embryon. Un autre inconvénient réside dans la mise en oeuvre relativement coûteuse d'une telle technique, induisant un frein à son développement industriel.

Une deuxième technique connue est décrite dans la demande de brevet US2011/0144473, qui décrit l'utilisation de la spectroscopie UV. Cette technique consiste à émettre une onde dans l'ultraviolet de manière à provoquer une auto-fluorescence d'une région du germe (blastoderme). Pour déterminer le sexe de l'embryon, on calcule puis on compare un coefficient de décroissance à une base de données, suite à l'observation de la décroissance de l'auto-fluorescence après arrêt de l'émission de l'onde UV. Cependant, un inconvénient de cette technique est qu'elle requiert un accès au blastoderme par un prélèvement invasif.

Une troisième technique connue est basée sur la spectroscopie infrarouge. Cette méthode, également invasive, consiste en l'introduction d'une sonde dans la région du germe, afin d'en caractériser les cellules constitutives par une spectroscopie infrarouge.

Un inconvénient majeur des deuxième et troisième techniques connues est qu'elles sont invasives : elles nécessitent l'introduction d'instruments et le prélèvement de cellules dans le germe. Ces opérations ne sont donc pas sans risques pour le développement futur de l'embryon. Un autre inconvénient de ces deux techniques est le risque de contamination de l'ensemble de la couvée.

Afin de pallier cet inconvénient majeur, une quatrième technique connue, non invasive, est décrite dans la demande de brevet US2013/0044210. Elle consiste en une analyse hyper-spectrale (c'est-à-dire de l'infrarouge moyen au proche UV) d'un spectre optique en réflexion de l'oeuf, en soustrayant la réponse spectrale de la coquille et en détectant les composants biologiques de l'oeuf. Cette technique permet de déterminer, dès le deuxième jour, si l'oeuf est fécondé et, au douzième jour, le genre de l'embryon. Un inconvénient majeur de cette technique est qu'elle nécessite des corrections pour s'affranchir de la coquille (soustraction de sa réponse spectrale) et des perturbations environnementales (lumière, humidité, etc.). À cet effet, chaque oeuf doit être posé sur un support spécifique, maintenant une distance connue entre la source de rayonnement, le détecteur et l'oeuf. Cette technique est donc difficilement industrialisable.

Du fait probablement des inconvénients précités, aucune des techniques connues citées précédemment n'a vraiment donné lieu à un développement industriel.

## 3. OBJECTIFS DE L'INVENTION

L'invention, dans au moins un mode de réalisation, a notamment pour objectif de pallier ces différents inconvénients de l'état de la technique.

Plus précisément, dans au moins un mode de réalisation de l'invention, un objectif est de fournir une technique de détermination de la fertilité et/ou du sexe d'un oeuf, qui soit non invasive (pas de pénétration de la coquille par un outil quelconque, ni aucun prélèvement d'échantillon dans l'oeuf) et plus simple à mettre en oeuvre, et donc plus facilement industrialisable, que la quatrième technique connue précitée.

Un autre objectif, dans au moins un mode de réalisation de l'invention est de fournir une telle technique qui soit fiable.

## 4. EXPOSÉ DE L'INVENTION

Ces objectifs, ainsi que d'autres qui apparaîtront plus clairement par la suite, sont atteints à l'aide d'un dispositif non invasif de détermination de la fertilité et/ou du sexe d'un oeuf,

Selon l'invention, ledit dispositif comprend des moyens d'obtention d'une réponse spectrale spécifique à la coquille à un signal lumineux incident, comprenant des moyens de focalisation adaptés pour focaliser ledit signal lumineux incident sur une portion de surface et/ou une portion interne d'au moins une couche de la coquille, et des moyens de détermination de la fertilité et/ou du sexe de l'oeuf en fonction de ladite réponse spectrale spécifique de la coquille.

Le principe général de l'invention consiste donc à obtenir une réponse spectrale spécifique à la coquille à un signal lumineux incident et à déterminer la fertilité et/ou le sexe de l'oeuf selon cette réponse spectrale.

Pour ce faire, l'invention met donc en oeuvre des moyens de focalisation particuliers permettant de focaliser le signal lumineux incident sur la surface et/ou sur une portion interne d'une couche bien précise de la coquille à examiner.

Ainsi, ce mode de réalisation particulier de l'invention repose sur une approche tout à fait nouvelle et inventive de détermination non invasive de la fertilité et/ou du sexe d'un oeuf, puisqu'elle s'appuie sur une spectroscopie de la surface et/ou d'une portion interne d'une couche de la coquille de l'oeuf. En effet, comme détaillé plus bas, les inventeurs ont émis l'hypothèse que les substances discriminantes, existant dans toutes les cellules de l'embryon et jouant un rôle dans la différenciation sexuelle, ne se concentraient pas exclusivement dans les gonades, mais se diffusent dans l'oeuf par l'intermédiaire du liquide allantoïdien et peuvent en partie infime mais mesurable, être présentes à la surface et à l'intérieur de la coquille.

On rappelle que dans la quatrième technique connue (seule technique non invasive connue des inventeurs), on cherche au contraire à s'affranchir de la réponse spectrale de la coquille en la soustrayant de celle de l'oeuf, pour ne conserver que la réponse spectrale du contenu de l'oeuf (comprenant l'albumine, le vitellus et le disque germinal).

Le signal lumineux incident est soit un signal lumineux mono-longueur d'onde (i.e. présentant une longueur d'onde unique), soit un signal lumineux multi-longueur d'onde, présentant au moins deux longueurs d'onde (successivement ou simultanément).

Selon une caractéristique particulière, le signal lumineux incident comprend au moins une longueur d'onde comprise entre 200 et 1100 nm.

Cette plage de longueurs d'onde permet un fonctionnement optimal.

Selon un aspect particulier, les moyens de focalisation sont adaptés pour focaliser le signal lumineux incident sur une portion de la surface extérieure d'une couche externe de la coquille.

L'avantage de focaliser sur la surface de la coquille est que le signal réfléchi (par exemple un signal Raman réfléchi) est plus riche, l'absorption étant moindre.

Selon un aspect particulier, les moyens de focalisation comprennent une sonde, possédant une distance de focalisation D par rapport à une lentille, et un embout dont une première extrémité est solidaire de la sonde et placée à une distance d1 de la lentille, et dont une seconde extrémité est destinée à être en contact avec l'oeuf, l'embout présentant une distance d2 entre les première et seconde extrémités, avec d2=D-d1.

Un tel embout permet donc à la sonde d'être toujours à la même distance de la surface de l'oeuf. En d'autres termes, l'embout permet une adaptabilité aisée du dispositif à toutes tailles d'oeufs, sans réglage supplémentaire. L'industrialisation d'un tel dispositif est donc naturellement envisageable.

La distance entre la lentille de la sonde et l'oeuf étant fixée par l'embout calibré en longueur, la distance de focalisation D peut aisément être réglée en modifiant la longueur d'onde du signal lumineux incident. Un tel aspect permet également une grande précision du dispositif. De plus, le réglage et la maintenance d'un tel positif sont facilités puisque seule la longueur d'onde est considérée comme variable.

Selon un aspect particulier, les moyens d'obtention de la réponse spectrale comprennent une sonde, ou ladite sonde, adaptée pour transmettre le signal lumineux incident vers l'oeuf et pour recevoir, avec un angle de réflexion nul, un signal lumineux réfléchi résultant d'une réflexion par l'oeuf du signal lumineux incident.

De cette façon, même si le signal lumineux réfléchi est relativement faible, une telle sonde permet d'assurer et d'optimiser la récupération du signal réfléchi.

Dans un mode particulier de l'invention, la sonde est une sonde de type Raman.

Une telle sonde permet une grande précision dans la transmission du signal incident et dans la réception du signal réfléchi.

Dans un mode particulier de l'invention, les moyens d'obtention de la réponse spectrale spécifique à la coquille comprennent :

- une sonde à réflexion totale atténuée, comprenant un cristal, qui reçoit un signal lumineux incident et génère un signal lumineux réfléchi ;
- un spectromètre qui récupère le signal lumineux réfléchi généré par ladite sonde ; et
- des moyens de traitement configurés pour obtenir la réponse spectrale spécifique à la coquille en fonction dudit signal lumineux incident et dudit signal lumineux réfléchi.

De tels moyens d'obtention de la réponse spectrale spécifique à la coquille permettent à la sonde d'être toujours à la même distance de la surface de l'oeuf puisque le cristal des sondes ATR est en contact avec l'échantillon à étudier. Cet aspect permet une adaptabilité aisée du dispositif à toutes tailles d'oeufs, sans réglage supplémentaire.

Lors de cette spectroscopie, une partie du signal incident est absorbée par la coquille atténuant ainsi le signal réfléchi. La réponse spectrale du signal incident étant connue et la réponse spectrale du signal incident étant mesurée précisément par le spectromètre, la réponse spectrale spécifique à la coquille de l'oeuf peut être déduite aisément par les moyens de traitement en soustrayant la réponse spectrale du signal réfléchi à la réponse spectrale du signal incident. En d'autres termes, la réponse spectrale spécifique à la coquille correspond à la réponse spectrale du signal atténué par la coquille.

Selon un aspect particulier de l'invention, les moyens de détermination de la fertilité et/ou du sexe de l'oeuf comprennent des moyens de comparaison de la réponse spectrale obtenue avec au moins une réponse spectrale de référence.

Une telle comparaison permet de faciliter la détermination de la fertilité et/ou du sexe de l'oeuf. En effet, les moyens de comparaison permettent d'éviter de déterminer précisément quelles sont les substances discriminantes présentes, mais de comparer l'allure générale du spectre obtenu avec un ou plusieurs spectres de référence.

Selon un aspect particulier de l'invention, ladite au moins une réponse spectrale de référence appartient au groupe comprenant :

- une première réponse spectrale de référence pour des oeufs non fécondés ;
- une deuxième réponse spectrale de référence pour des oeufs fécondés et contenant un embryon mort ;
- une troisième réponse spectrale de référence pour des oeufs fécondés et contenant un embryon mâle ; et
- une quatrième réponse spectrale de référence pour des oeufs fécondés et contenant un embryon femelle.

L'invention concerne également un procédé non invasif de détermination de la fertilité et/ou du sexe d'un oeuf, comprenant une étape d'obtention d'une réponse spectrale d'au moins une portion d'au moins une couche de la coquille à un signal lumineux incident, et une étape de détermination de la fertilité et/ou du sexe de l'oeuf en fonction de la réponse spectrale obtenue.

## 5. LISTE DES FIGURES

D'autres caractéristiques et avantages de l'invention apparaîtront à la lecture de la description suivante, donnée à titre d'exemple indicatif et non limitatif, et des dessins annexés, dans lesquels :

- la figure 1 présente la structure simplifiée d'un oeuf fécondé ;
- la figure 2 est une vue en coupe de la structure de la coquille d'un oeuf ;
- la figure 3 présente un dispositif de détermination de la fertilité et/ou du sexe d'un oeuf, selon un premier mode de réalisation de l'invention ;
- la figure 4 est une vue de détail du fonctionnement de la sonde du dispositif de la figure 3 ; et
- la figure 5 illustre un exemple de spectre obtenu avec le dispositif de la figure 3;
- la figure 6 présente un dispositif de détermination de la fertilité et/ou du sexe d'un oeuf, selon un deuxième mode de réalisation de l'invention ; et
- la figure 7 est une vue de détail du fonctionnement de la sonde du dispositif de la figure 6.

## 6. DESCRIPTION DÉTAILLÉE DE DEUX MODES DE REALISATION

*6.1 Prérequis*

Sur toutes les figures du présent document, les éléments et étapes identiques sont désignés par une même référence numérique.

Les inventeurs sont partis de plusieurs constats, à savoir :

1. La différenciation sexuelle de l'embryon ne résulte pas essentiellement de l'action d'hormones sur les gonades.
2. Les cellules somatiques ont une identité sexuelle autonome.
3. Les principales substances participant à la détermination femelle comprennent :

a. des protéines telle que la FOXL2 impliquée dans le maintien de la fonction ovarienne ;
b. des oestrogènes comme par exemple l'estradiol secrété par l'ovaire de l'embryon ;
c. des enzymes tels que la MHM ou l'Aromatase (CYP19A1) qui aide à la conversion d'hormones stéroïdiennes en oestrogène ;
d. du matériel génétique tel que le gène HINTW (ou WPKCI) présent dans plusieurs tissus de l'embryon femelle ainsi que les gènes DAX1, WNT4, FET1.

4. Les principales substances participant à la détermination mâle comprennent :

a. des hormones, comme par exemple l'AMH responsable de la régression des voies génitales supérieures femelles ;
b. du matériel génétique, tel que le gène DMRT1 qui participe à la formation des testicules ou le SOX9 participant à la formation des gonades.

Les substances précitées (aussi appelées par la suite « substances discriminantes ») sont de différents types :

- des produits de dégradation issus de la biosynthèse, comme l'aromatase qui indique la présence de stéroïdes, l'hormone AMH présente dans le liquide allantoïdien en plus grande quantité pour l'embryon femelle que pour le mâle, l'enzyme MHM ou le gène HINTW ;
- des stéroïdes, comme l'estradiole sont également présents dans le liquide allantoïdien.

Les inventeurs ont émis l'hypothèse que ces substances discriminantes, existant dans toutes les cellules de l'embryon et jouant un rôle dans la différenciation sexuelle, ne se concentraient pas exclusivement dans les gonades, mais se diffusent dans l'oeuf par l'intermédiaire du liquide allantoïdien et peuvent en partie infime mais mesurable, soit migrer vers et à travers la coquille (lors de l'évacuation de la vapeur d'eau ou du CO2), soit s'incruster dans la coquille. Selon leur taille, elles se retrouvent donc soit à la surface de la coquille, soit incrustées dans la coquille (par exemple dans les membranes coquillières).

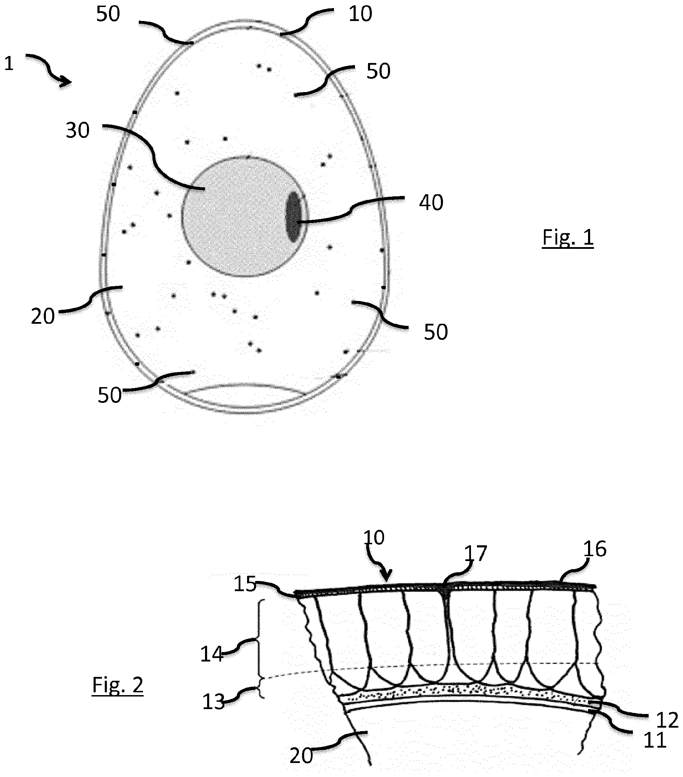

Comme illustré sur la **figure 1,** un oeuf 1 comprend une coquille 10 et un contenu intérieur comprenant lui-même l'albumine 20, le vitellus 30 et le disque germinal 40.

Comme illustré sur la **figure 2,** la coquille 10 comprend une superposition de six couches, à savoir (en partant de l'albumine 20) : la membrane coquillère interne 11, la membrane coquillère externe 12, la couche mamillaire 13, la couche palissade 14 traversée par les pores 17, une monocouche de cristaux verticaux (15) et enfin la cuticule 16 enveloppant l'ensemble des couches minérales.

Comme indiqué plus haut, et illustré sur la figure 1, une pluralité de substances discriminantes 50 sont présentes non seulement dans le contenu intérieur (et plus précisément dans l'albumine 20), mais aussi à la surface et/ou à l'intérieur de la coquille 10. Bien que faible, la présence et la quantité de ces substances discriminantes 50 à la surface et/ou dans la coquille 10 sont alors mesurables par spectroscopie.

*6.2 Description d'un premier mode de réalisation*

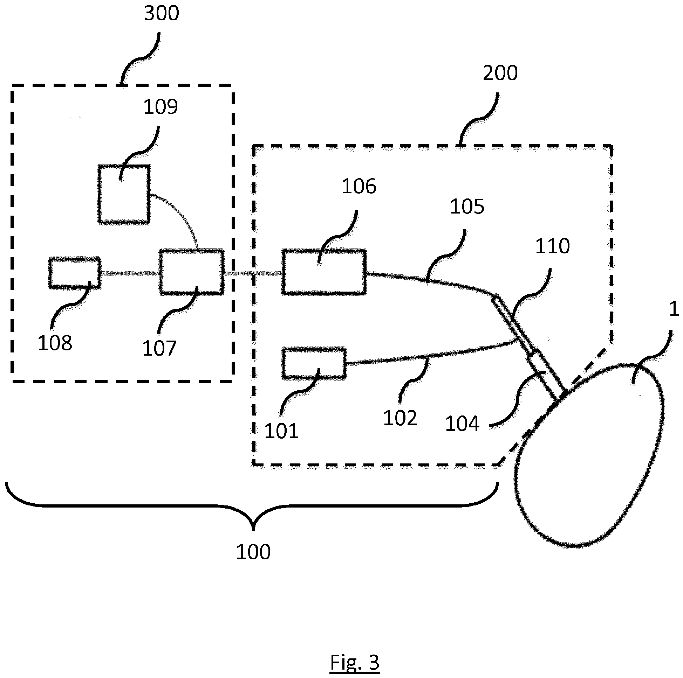

On présente maintenant, en relation avec la **figure 3,** un dispositif 100 de détermination de la fertilité et/ou du sexe d'un oeuf, selon un premier mode de réalisation particulier de l'invention.

La spectroscopie Raman est une technique d'analyse non destructive permettant de caractériser la composition moléculaire et la structure d'un matériau. Cette technique est basée sur la détection des photons diffusés inélastiquement suite à l'interaction de l'échantillon avec un faisceau de lumière monochromatique (diffusion Raman). La différence de fréquence entre le photon excitateur et le photon réfléchi renseigne sur la nature chimique de la substance (molécule) à l'origine de la diffusion. Autrement dit, la diffusion Raman est le phénomène physique par lequel un milieu est capable de modifier légèrement la fréquence de la lumière qui y circule. Ce décalage en fréquence correspond à un échange d'énergie entre le rayon lumineux et le milieu. Cet échange peut avoir plusieurs causes : vibrations du cristal ou de la molécule, excitations magnétiques, etc. La mesure de ce décalage permet de remonter à certaines propriétés du milieu.

Dans le mode de réalisation particulier décrit ci-après, on utilise la spectroscopie Raman afin de déterminer la fertilité et/ou le sexe d'un oeuf. Il est clair cependant que la présente invention n'est pas limitée à ce type de spectroscopie, mais peut être mise en oeuvre avec toute technique permettant d'obtenir la réponse spectrale d'au moins une portion d'au moins une couche de la coquille. Le second mode de réalisation, décrit plus bas, en est un exemple.

Le principe général de la technique proposée est le suivant : une faible surface de la coquille reçoit au moins une onde électromagnétique incidente de longueur définie. Selon la technique employée, le spot généré (aussi appelé ci-après « point ou zone de focalisation ») aura par exemple un diamètre de quelques microns à 150 microns.

L'onde incidente est réfléchie à la même fréquence pour la plus grande part, et à des fréquences différentes pour une partie infime. Selon le type de spectroscopie employée, on exploite l'une ou l'autre des ondes réfléchies.

Dans le mode de réalisation présenté, le dispositif 100 comprend :

- des moyens 200 d'obtention d'une réponse spectrale spécifique à la coquille 10 d'un oeuf 1. Ces moyens 200 comprennent une source de lumière 101, une première fibre optique 102, une sonde 110, un embout 104, une deuxième fibre optique 105 et un spectromètre 106 ;
- des moyens 300 de détermination de la fertilité et/ou du sexe de l'oeuf 1 en fonction de la réponse spectrale spécifique à la coquille 10. Ces moyens 300 comprennent un calculateur 107, une base de données 109 et un afficheur 108.

La source de lumière 101 est par exemple un laser, apte à fournir un signal lumineux incident. Plus particulièrement, le signal lumineux fourni par le laser 101 est par exemple une onde électromagnétique de longueur d'onde unique, préférentiellement située entre 200 et 1100 nm (et encore plus préférentiellement entre 500 et 700nm).

Dans une variante (spectroscopie multi-longueur d'onde), le signal lumineux incident est multi-longueur d'onde, avec au moins deux longueurs d'onde (successives ou simultanées) comprises chacune entre 200 et 1100 nm. Cette variante permet d'obtenir un spectre plus complet, et par conséquent des résultats plus fiables. Elle permet également de s'affranchir de la fluorescence.

La sonde 110 et l'embout 104 forment ensemble des moyens de focalisation. La première fibre optique 102 est destinée à transmettre le signal lumineux incident à la sonde 110.

L'embout 104 est calibré en longueur et solidaire de la sonde 110. Il permet à la sonde 110 d'être située à distance constante de l'oeuf 1. Une première extrémité de l'embout 104 est solidaire de la sonde 110, et une deuxième extrémité de l'embout 104 est destinée à venir en contact avec l'oeuf 1.

La réponse du signal lumineux réfléchi par la surface de la coquille 10 de l'oeuf 1 étant relativement faible, la sonde 110 est, dans un mode de réalisation particulier, adaptée pour récupérer le signal réfléchi dans le même axe que le signal incident. Ce type de sonde est plus communément connu sous le nom de sonde Raman.

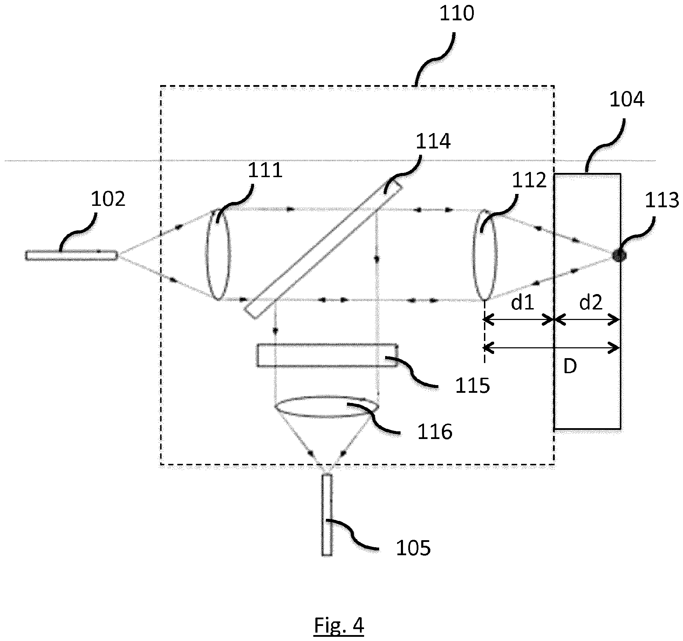

Le principe de fonctionnement d'une telle sonde Raman 110 est illustré **figure 4.** Le signal lumineux incident issu de la première fibre optique 102 est collimaté au moyen d'une première lentille 111. Une lumière collimatée est une lumière dont les rayonnements sont sensiblement parallèles et se déploient lentement quand ils se propagent. En d'autres mots, c'est une lumière qui ne se disperse pas avec la distance (en théorie), ou qui sera très peu dispersée (en pratique). Ce signal collimaté est ensuite focalisé, par l'intermédiaire d'une deuxième lentille 112, en un point ou une zone de focalisation 113, situé(e) à la surface ou à l'intérieur de la coquille 10 à examiner. Le point de focalisation 113 est aussi appelé spot.

Les première et deuxième lentilles (111, 112) étant fixes, la distance de focalisation ne peut être modifiée que par variation de la longueur d'onde du signal lumineux incident. On définit ici la distance de focalisation D de la sonde 110 comme la distance entre la deuxième lentille 111 et le spot 113.

Dans une implémentation particulière (illustrée sur la figure 3), le spot 113 se situe sur la surface extérieure de la couche 16 externe de la coquille 10. A cet effet, la longueur d2 de l'embout 104 est obtenue selon la formule : d2=D-d1, avec d1 la distance séparant une première extrémité de l'embout 104 (solidaire de la sonde 104) et la lentille 112. Une deuxième extrémité de l'embout 104, situé à la distance d2 de la première extrémité, est destinée à être en contact avec la surface externe de l'oeuf.

Dans cette implémentation particulière, le signal lumineux incident est réfléchi (par la portion de la surface de l'oeuf qui correspond au spot 113) et le signal réfléchi est collecté/collimaté par la même lentille 112, puis dirigé au moyen d'un miroir dichroïque 114 vers le spectromètre 106.

Afin d'optimiser le signal reçu par le spectromètre 106, le signal dirigé au moyen d'un miroir dichroïque 114 passe au préalable à travers un filtre optique 115 passe-haut, destiné à éliminer la diffusion Rayleigh, est ensuite focalisé par une troisième lentille 116, et est enfin transmis au spectromètre 106 par la deuxième fibre optique 105.

Le spectromètre 106 analyse le signal reçu et fournit une réponse spectrale aux moyens 300 de détermination de la fertilité et/ou du sexe de l'oeuf 1.

Au sein des moyens 300 de détermination de la fertilité et/ou du sexe de l'oeuf 1, le calculateur 107 et la base de données 109 forment des moyens de comparaison. Ces derniers permettent d'exploiter la réponse spectrale transmise par le spectromètre 106 et de la comparer avec une bibliothèque de réponses spectrales de référence. Cette comparaison porte soit sur l'amplitude d'une ou plusieurs raies de la réponse spectrale, soit sur l'allure de l'ensemble de la réponse spectrale.

Dans une mise en oeuvre particulière, on utilise les quatre réponses spectrales de référence suivantes :

- une première réponse spectrale de référence, pour les oeufs non fécondés ;
- une deuxième réponse spectrale de référence, pour les oeufs fécondés et contenant un embryon mort ;
- une troisième réponse spectrale de référence, pour les oeufs fécondés et contenant un embryon mâle ;
- une quatrième réponse spectrale de référence, pour les oeufs fécondés et contenant un embryon femelle.

Dans une variante, le groupe des oeufs non fécondés et celui des oeufs fécondés mais contenant un embryon mort peuvent être regroupés en un seul et même groupe. En d'autres termes, les première et quatrième réponses spectrales de référence sont une seule et même réponse spectrale de référence.

L'afficheur 108 permet d'afficher la réponse spectrale obtenue, ainsi que le résultat de la comparaison de celle-ci avec la base de données des réponses spectrales de référence.

L'obtention de la base de données 109 comprend par exemple les étapes suivantes :

• Etape 1 : sur une couvée (« couvée de configuration »), un spectre Raman (réponse spectrale de la coquille selon la technique décrite plus haut en relation avec les figures 3 et 4) est relevé sur chaque oeuf à différents jours d'incubation ;
• Etape 2 : une corrélation est réalisée afin de déterminer les zones qui diffèrent d'un spectre à l'autre (zones de détermination). Cette méthode permet de classer les spectres en catégories (réponses spectrales de référence).
• Etape 3 : le sexage des poussins permet ensuite de classer ces catégories, c'est-à-dire d'associer à chaque catégorie l'une des informations suivantes : « embryon mâle », « embryon femelle » et « oeuf clair ou embryon mort ».

Les étapes 1 à 3 peuvent être réitérées sur plusieurs « couvées de configuration ». La base de données 109 est ainsi enrichie par l'apport régulier de nouvelles mesures.

L'algorithme de décision quant à la fertilité et/ou au sexe d'un oeuf comprend par exemple les étapes suivantes, pour chaque oeuf d'une couvée donnée (« couvée à tester ») :

- Etape a : un spectre Raman (réponse spectrale de la coquille selon la technique décrite plus haut en relation avec les figures 3 et 4) est relevé sur l'oeuf à tester;
- Etape b : la qualité de la mesure est évaluée sur chaque zone de détermination (cf. définition plus haut), par exemple en calculant la dérivée première et la dérivée seconde du spectre. Le critère de qualité est par exemple calculé en prenant en compte la dynamique de mesure représentée par la déviation maximale de la mesure dans la zone, la variabilité des données représentée par le nombre d'inversion de pente dans la zone donnée par la dérivée première et le niveau de bruit donné par l'écart-type de la dérivée seconde dans la zone.
- Etape c : si le critère de qualité est suffisant, le spectre est comparé, par corrélation sur les zones de détermination, avec les différents spectres de référence de la base de données, afin d'en déterminer la catégorie et donc le genre (parmi « embryon mâle », « embryon femelle » et « oeuf clair ou embryon mort »).

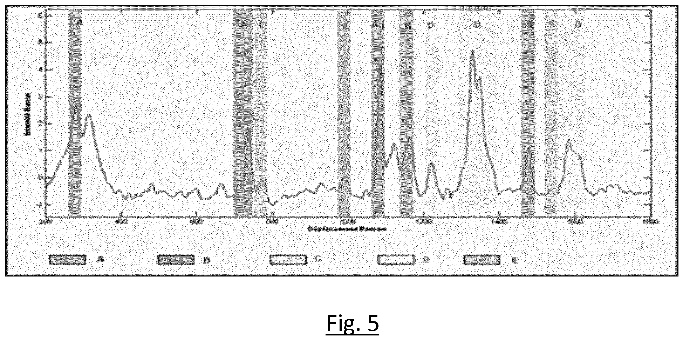

La **figure 5** illustre un exemple de réponse spectrale (spectre) spécifique à la coquille 10 obtenue avec le dispositif 100 de la figure 3. Ce spectre est représentatif d'une combinaison des réponses des substances 50 présentes à la surface et/ou à l'intérieur de la coquille 10 de l'oeuf 1. La comparaison de ces spectres avec les spectres d'échantillons biologiques référencés dans la littérature permet d'associer aux bandes visibles la présence de certains composés ou molécules. À titre d'exemple, on associe les bandes A au carbonate de calcium, les bandes B aux caroténoïdes, C à l'ADN, D aux structures lipidiques et protéiques et E à la phénylalanine. La comparaison à la base de données s'effectue sur ces bandes mais également sur des bandes intermédiaires où les signaux sont plus faibles comme dans la zone 800 à 900 $cm^{-1}$.

Dans une variante de ce mode de réalisation, la longueur d2 de l'embout 104 est calculée de façon que le point de focalisation 113 soit situé dans l'une des couches de la coquille 10. En d'autres mots, on recherche la présence des substances discriminantes 50 non plus à la surface extérieure de la couche externe 16 de la coquille 10, mais à l'intérieur de l'une des couches (11, 12, 13, 14, 15 et 16) formant la coquille 10.

*6.3 Description d'un deuxième mode de réalisation*

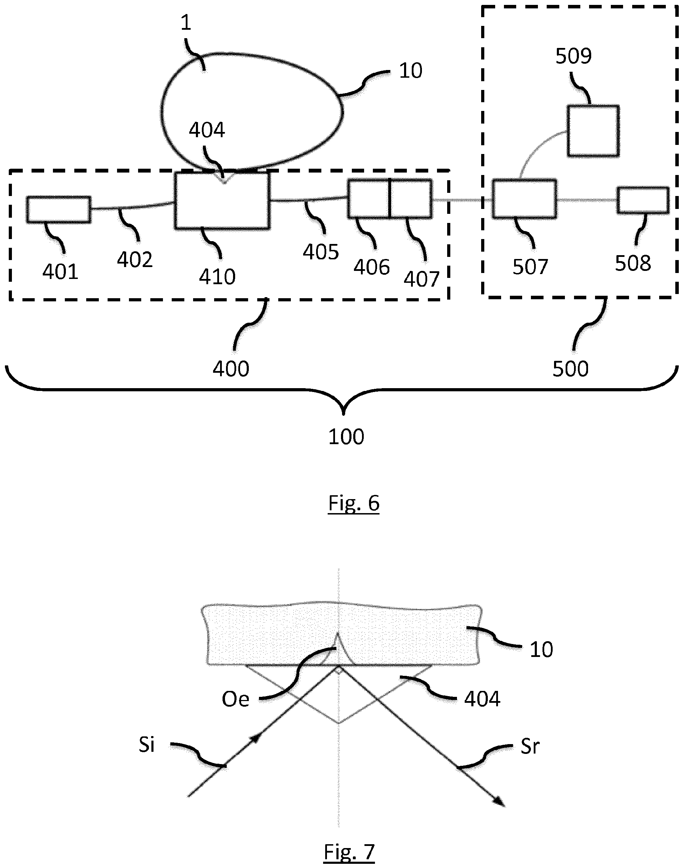

La figure 6 illustre un deuxième mode de réalisation dans lequel la détermination de la fertilité et/ou du sexe de l'oeuf est effectuée par le biais d'une spectroscopie infrarouge à réflexion totale atténuée, dite « spectroscopie ATR ».

Dans ce deuxième mode de réalisation, le dispositif 100 comprend :

- des moyens 400 d'obtention d'une réponse spectrale d'au moins une portion d'au moins une couche de la coquille 10 d'un oeuf 1. Ces moyens 400 comprennent une source de lumière 401, une première fibre optique 402, une sonde 410, un cristal 404, une deuxième fibre optique 405, un spectromètre 406 et des moyens de traitement 407 ;
- des moyens 500 de détermination de la fertilité et/ou du sexe de l'oeuf 1 en fonction de la réponse spectrale spécifique à la coquille 10. Ces moyens 500 comprennent un calculateur 507, une base de données 509 et un afficheur 508.

La source de lumière 401 est une source polychromatique, comme par exemple un lasers au dioxyde de carbone ou les filaments de carbure de silicium chauffés, apte à fournir un signal lumineux incident dont les longueurs d'ondes se situent dans l'infrarouge ou le proche infrarouge.

La sonde 410 et le cristal 404 forment ensemble des moyens de focalisation. La première fibre optique 402 est destinée à transmettre le signal lumineux incident à la sonde 410.

La sonde 410 est une sonde de type ATR (abréviation de réflexion totale atténuée) et comprend un cristal 404 transparent aux infrarouges. Le cristal 404 présente un indice de réflexion/réfraction élevé, ou dans tous les cas supérieur à l'indice de réfraction de l'échantillon (ici la coquille 10 de l'oeuf 1).

Le cristal 404 est destiné à venir en contact avec la coquille 10 de l'oeuf et permet donc à la sonde 410 d'être située à distance constante de l'oeuf 1.

Comme illustré sur les figures 6 et 7, le signal lumineux incident Si est transmis par la première fibre optique 402 jusqu'à la sonde 410. Le signal lumineux incident Si traverse le cristal et est, en théorie, totalement réfléchi par l'extrémité du cristal. Le signal lumineux réfléchi Sr est ensuite transmis, par le biais de la deuxième fibre optique 405 vers le spectromètre 406.

Toutefois, en pratique, ce phénomène de réflexion est perturbé par la formation d'une onde évanescente Oe qui se crée lors du contact du signal lumineux incident Si avec la coquille 10. En effet, le signal lumineux incident Si est en partie absorbé par la coquille 10 atténuant ainsi le signal lumineux réfléchi Sr.

La réponse spectrale obtenue par le spectromètre 406 doit ensuite être traitée par les moyens de traitement 407 afin d'obtenir la réponse spectrale spécifique à la coquille 10, c'est-à-dire la réponse spectrale ROe de l'onde évanescente Oe.

La réponse spectrale RSi du signal lumineux incident Si et la réponse spectrale RSr du signal lumineux réfléchi Sr étant connues, il suffit donc de déduire la réponse spectrale ROe de l'onde évanescente Oe. Pour cela, il suffit de soustraire la réponse spectrale du signal réfléchi de la réponse spectrale du signal incident, ainsi :

$$ROe = RSi\text{-}RSr$$

La réponse spectrale ROe de l'onde évanescente Oe est équivalente/représentative de la réponse spectrale de la coquille 10.

Les moyens de traitement 407 fournissent ensuite la réponse spectrale spécifique à la coquille aux moyens 500 de détermination de la fertilité et/ou du sexe de l'oeuf 1.

Au sein des moyens 500 de détermination de la fertilité et/ou du sexe de l'oeuf 1, le calculateur 507 et la base de données 509 forment des moyens de comparaison. Ces derniers permettent d'exploiter la réponse spectrale transmise par les moyens de traitement 407 et de la comparer avec une bibliothèque de réponses spectrales de référence. Cette comparaison porte soit sur l'amplitude d'une ou plusieurs raies de la réponse spectrale, soit sur l'allure de l'ensemble de la réponse spectrale.

Cette comparaison permet de détecter des substances chimiques discriminantes présentent à la surface de la coquille 10 afin de déterminer la fertilité et/ou le sexe de l'oeuf 1.

L'afficheur 508 permet d'afficher la réponse spectrale spécifique à la coquille, ainsi que le résultat de la comparaison de celle-ci avec la base de données des réponses spectrales de référence.

Plus généralement, le calculateur 507, la base de données 509 et l'afficheur 508 présentent un structure et un fonctionnement identiques au calculateur 107, à la base de données 109 et à l'afficheur 108 décrits plus en détails dans le premier mode de réalisation.

## Revendications

**1.** Dispositif non invasif de détermination de la fertilité et/ou du sexe d'un oeuf (1), **caractérisé en ce qu'**il comprend des moyens (200 ; 400) d'obtention d'une réponse spectrale spécifique à la coquille (10) à un signal lumineux incident, comprenant des moyens (110, 104 ; 410, 404) de focalisation configurés pour focaliser ledit signal lumineux incident sur une portion de surface (113) et/ou une portion interne d'au moins une couche de la coquille (10), et des moyens (300 ; 500) de détermination de la fertilité et/ou du sexe de l'oeuf (1) en fonction de ladite réponse spectrale spécifique à la coquille.

**2.** Dispositif selon la revendication 1, **caractérisé en ce que** le signal lumineux incident comprend au moins une longueur d'onde comprise entre 200 et 1100 nm.

**3.** Dispositif selon l'une quelconque des revendications 1 et 2, **caractérisé en ce que** les moyens de focalisation (110, 104 ; 410, 404) sont adaptés pour focaliser le signal lumineux incident sur une portion de la surface extérieure d'une couche externe (16) de la coquille (10).

**4.** Dispositif selon la revendication 3, **caractérisé en ce que** les moyens de focalisation (110, 104) comprennent une sonde (110), possédant une distance de focalisation D par rapport à une lentille (112), et un embout (104) dont une première extrémité est solidaire de la sonde et placée à une distance d1 de la lentille (112), et dont une seconde extrémité est destinée à être en contact avec l'oeuf, l'embout présentant une distance d2 entre les première et seconde extrémités, avec d2=D-d1.

**5.** Dispositif selon l'une quelconque des revendications 1 à 4, **caractérisé en ce que** les moyens (200) d'obtention de la réponse spectrale comprennent une sonde (110), ou ladite sonde, configurée pour transmettre le signal lumineux incident vers l'oeuf (1) et pour recevoir, avec un angle de réflexion nul, un signal lumineux réfléchi résultant d'une réflexion par l'oeuf (1) du signal lumineux incident.

**6.** Dispositif selon la revendication 5, **caractérisé en ce que** la sonde (110) est une sonde de type Raman.

**7.** Dispositif selon l'une quelconque des revendications 1 à 3, **caractérisé en ce que** les moyens d'obtention (400) de la réponse spectrale spécifique à la coquille (10) comprennent :

- une sonde (410) à réflexion totale atténuée, comprenant un cristal (404), qui reçoit un signal lumineux incident (Si) et génère un signal lumineux réfléchi (Sr) ;
- un spectromètre (406) qui récupère le signal lumineux réfléchi (Sr) généré par ladite sonde (410) ; et
- des moyens de traitement (407) configurés pour obtenir la réponse spectrale spécifique à la coquille (10) en fonction dudit signal lumineux incident (Si) et dudit signal lumineux réfléchi (Sr).

**8.** Dispositif selon l'une quelconque des revendications 1 à 7, **caractérisé en ce que** les moyens (300 ; 500) de détermination de la fertilité et/ou du sexe de l'oeuf (1) comprennent des moyens de comparaison (107, 109 ; 507, 509) de la réponse spectrale obtenue avec au moins une réponse spectrale de référence.

9. Dispositif selon la revendication 8, **caractérisé en ce que** ladite au moins une réponse spectrale de référence appartient au groupe comprenant :

   - une première réponse spectrale de référence pour des oeufs non fécondés ;
   - une deuxième réponse spectrale de référence pour des oeufs fécondés et contenant un embryon mort ;
   - une troisième réponse spectrale de référence pour des oeufs fécondés et contenant un embryon mâle ; et
   - une quatrième réponse spectrale de référence pour des oeufs fécondés et contenant un embryon femelle.

10. Procédé non invasif de détermination de la fertilité et/ou du sexe d'un oeuf (1), **caractérisé en ce qu'**il comprend une étape d'obtention d'une réponse spectrale spécifique à la coquille à un signal lumineux incident focalisé sur une portion de surface (113) et/ou une portion interne d'au moins une couche de la coquille (10), et une étape de détermination de la fertilité et/ou du sexe de l'oeuf (1) en fonction de ladite réponse spectrale spécifique à la coquille.

**Patentansprüche**

1. Vorrichtung zur nicht invasiven Bestimmung der Fruchtbarkeit und/oder des Geschlechts eines Eis (1), **dadurch gekennzeichnet, dass** sie Mittel (200; 400) zum Erhalt einer der Eierschale (10) spezifischen Spektralantwort auf ein eintreffendes Lichtsignal umfasst, umfassend Fokussierungsmittel (110, 104; 410, 404), die ausgeführt sind, um das eintreffende Lichtsignal auf einen Oberflächenabschnitt (113) und/oder einen Innenabschnitt mindestens einer Schicht der Eierschale (10) zu fokussieren, und Mittel (300; 500) zur Bestimmung der Fruchtbarkeit und/oder des Geschlechts des Eis (1) in Abhängigkeit von der der Eierschale spezifischen Spektralantwort.

2. Vorrichtung nach Anspruch 1, **dadurch gekennzeichnet, dass** das eintreffende Lichtsignal mindestens eine Wellenlänge zwischen 200 und 1100 nm umfasst.

3. Vorrichtung nach einem der Ansprüche 1 und 2, **dadurch gekennzeichnet, dass** die Fokussierungsmittel (110, 104; 410, 404) eingerichtet sind, um das eintreffende Lichtsignal auf einen Abschnitt der Außenfläche einer äußeren Schicht (16) der Eierschale (10) zu fokussieren.

4. Vorrichtung nach Anspruch 3, **dadurch gekennzeichnet, dass** die Fokussierungsmittel (110, 104) eine Sonde (110), die eine Fokussierungsdistanz D zu einer Linse (112) besitzt, und einen Ansatz (104) umfassen, dessen erstes Ende mit der Sonde verbunden und in einer Distanz d1 zur Linse (112) angeordnet ist, und dessen zweites Ende dazu bestimmt ist, mit dem Ei in Kontakt zu sein, wobei der Ansatz eine Distanz d2 zwischen den ersten und zweiten Enden aufweist, wobei d2 = D - d1.

5. Vorrichtung nach einem der Ansprüche 1 bis 4, **dadurch gekennzeichnet, dass** die Mittel (200) zum Erhalt der Spektralantwort eine Sonde (110) oder die Sonde umfassen, die ausgeführt ist, um das eintreffende Lichtsignal zu dem Ei (1) zu übertragen, und um mit einem Reflexionswinkel gleich Null ein reflektiertes Lichtsignal zu empfangen, das sich aus einer Reflexion des eintreffenden Lichtsignals durch das Ei (1) ergibt.

6. Vorrichtung nach Anspruch 5, **dadurch gekennzeichnet, dass** die Sonde (110) eine Raman-Sonde ist.

7. Vorrichtung nach einem der Ansprüche 1 bis 3, **dadurch gekennzeichnet, dass** die Mittel (400) zum Erhalt der der Eierschale (10) spezifischen Spektralantwort umfassen:

   - eine Sonde (410) gedämpfter Totalreflexion, umfassend einen Kristall (404), der ein eintreffendes Lichtsignal (Si) empfängt und ein reflektiertes Lichtsignal (Sr) erzeugt;
   - ein Spektrometer (406), das das reflektierte Lichtsignal (Sr), das von der Sonde (410) erzeugt wird, wiederherstellt; und
   - Bearbeitungsmittel (407), die ausgeführt sind, um die der Eierschale (10) spezifische Spektralantwort in Abhängigkeit von dem eintreffenden Lichtsignal (Si) und dem reflektierten Lichtsignal (Sr) zu erhalten.

8. Vorrichtung nach einem der Ansprüche 1 bis 7, **dadurch gekennzeichnet, dass** die Mittel (300; 500) zur Bestimmung der Fruchtbarkeit und/oder des Geschlechts des Eis (1) Mittel (107, 109; 507, 509) zum Vergleich der erhaltenen Spektralantwort mit mindestens einer Referenzspektralantwort umfassen.

9. Vorrichtung nach Anspruch 8, **dadurch gekennzeichnet, dass** die mindestens eine Referenzspektralantwort der Gruppe angehört, umfassend:

   - eine erste Referenzspektralantwort für nicht befruchtete Eier;
   - eine zweite Referenzspektralantwort für befruchtete Eier, die einen toten Embryo enthalten;

- eine dritte Referenzspektralantwort für befruchtete Eier, die einen männlichen Embryo enthalten; und

- eine vierte Referenzspektralantwort für befruchtete Eier, die einen weiblichen Embryo enthalten.

10. Verfahren zur nicht invasiven Bestimmung der Fruchtbarkeit und/oder des Geschlechts eines Eis (1), **dadurch gekennzeichnet, dass** es einen Schritt des Erhalts einer der Eierschale spezifischen Spektralantwort auf ein eintreffendes Lichtsignal, das auf einen Oberflächenabschnitt (113) und/oder einen Innenabschnitt mindestens einer Schicht der Eierschale (10) fokussiert ist, und einen Schritt der Bestimmung der Fruchtbarkeit und/oder des Geschlechts des Eis (1) in Abhängigkeit von der der Eierschale spezifischen Spektralantwort umfasst.

**Claims**

1. Non-invasive device for determining the fertility and/or sex of an egg (1), **characterised in that** it comprises means (200; 400) for obtaining a spectral response, specific to the shell (10), to an incident light signal, comprising focusing means (110, 104; 410, 404) configured to focus said incident light signal on a surface portion (113) and/or an internal portion of at least one layer of the shell (10), and means (300; 500) for determining the fertility and/or sex of the egg (1) as a function of said spectral response specific to the shell.

2. Device according to Claim 1, **characterised in that** the incident light signal comprises at least one wavelength ranging from 200 and 1100 nm.

3. Device according to any one of the Claims 1 and 2, **characterised in that** the focusing means (110, 104; 410, 404) are adapted to focus the incident light signal on a portion of the outer surface of an external layer (16) of the shell (10).

4. Device according to Claim 3, **characterised in that** the focusing means (110, 104) comprise a probe (110) having a focusing distance D relative to a lens (112) and an end-piece (104), a first extremity of which is fixedly attached to the probe and placed at a distance d1 from the lens (112), and a second extremity of which is intended to be in contact with the egg, the end-piece having a distance d2 between the first and second extremities with d2 = D - d1.

5. Device according to any one of Claims 1 to 4, **characterised in that** the means (200) for obtaining the spectral response comprise a probe (110), or said probe, is configured to transmit the incident light signal towards the egg (1) and to receive, at a zero angle of reflection, a reflected light signal resulting from a reflection by the egg (1) of the incident light signal.

6. Device according to Claim 5, **characterised in that** the probe (110) is a Raman probe.

7. Device according to any one of Claims 1 to 3, **characterised in that** the means (400) for obtaining the spectral response specific to the shell (10) comprise:

- an attenuated total reflection probe (410) comprising a crystal (404), that receives an incident light signal (Si) and generates a reflected light signal (Sr);
- a spectrometer (406) which recovers the reflected light signal (Sr) generated by said probe (410); and
- processing means (407) configured to obtain the spectral response specific to the shell (10) as a function of said incident light signal (Si) and of said reflected light signal (Sr).

8. Device according to any one of Claims 1 to 7, **characterised in that** the means (300; 500) for determining the fertility and/or sex of the egg (1) comprise means (107, 109; 507, 509) for comparing the spectral response obtained with at least one reference spectral response.

9. Device according to Claim 8, **characterised in that** the said at least one reference spectral response belongs to the group comprising:

- a first reference spectral response for unfertilised eggs;
- a second reference spectral response for fertilised eggs containing a dead embryo;
- a third reference spectral response for fertilised eggs containing a male embryo; and
- a fourth reference spectral response for fertilised eggs containing a female embryo.

10. Non-invasive method for determining the fertility and/or sex of an egg (1), **characterised in that** it comprises a step for obtaining a spectral response, specific to the shell, to an incident light signal focused on a surface portion (113) and/or an internal portion of at least one layer of the shell (10), and a step for determining the fertility and/or sex of the egg (1) as a function of said spectral response specific to the shell.

50    10

1

30

40

_Fig. 1_

20

50

50

50

10    17    16

15

14

_Fig. 2_

13

12

11

20

Fig. 3

Fig. 4

Fig. 5

Fig. 6

Fig. 7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6029080 A
- US 20110144473 A
- US 20130044210 A